# EUROPÄISCHE PATENTSCHRIFT

(11) **EP 0 352 784 B1**
(45) Veröffentlichungstag und Bekanntmachung des Hinweises auf die Patenterteilung: **02.03.1994**
(21) Anmeldenummer: 89113850.5
(22) Anmeldetag: 27.07.1989
(51) Int. Cl.: C12N 9/10, C12N 11/00, C12P 21/02

(54) **Gamma-Glutamylcysteintransferase, Verfahren zu deren Herstellung und deren Verwendung**
Gamma-glutamyl cystein transferase, process for its production and use of same
Gamma-glutamylcystéine transférase, son procédé de production et son utilisation

(30) Priorität: 28.07.1988 DE 3825677
(43) Veröffentlichungstag der Anmeldung: 31.01.1990
(73) Patentinhaber: INDENA S.p.A., 20141 Milano (IT)
(72) Erfinder: Grill, Erwin, Dr. c/o Michigan State University, East Lansing Michigan 48824 (US); Löffler, Susanne, Dr., D-8000 München 70 (DE); Winnacker, Ernst-Ludwig, Prof. Dr., D-8000 München 19 (DE); Zenk, Meinhard, Prof. Dr., D-8000 München 60 (DE)
(74) Vertreter: Bianchetti, Giuseppe

(56) Entgegenhaltungen:
- DE-A- 3 529 625
- BIOCHIMICA ET BIOPHYSICA ACTA, Band 399, 1975, Seiten 1-9, Amsterdam, NL; P. APONTOWEIL et al.: "Glutathione biosynthesis in Escherichia coli K 12 properties of the enzymes and regulation"

## Beschreibung

Die Erfindung betrifft ein katalytisch wirksames Protein. Es katalysiert den Transfer von γ-Glutamylcystein bzw. γ-Glutamylcystein-S-derivaten.

Gegenstand der Erfindung ist γ-Glutamylcysteintransferase.

Das Enzym wird durch folgende Parameter gekennzeichnet:
Molgewicht 95.000 ± 10%, bestimmt durch Gelfiltration; Enzym nativ vorliegend als dimeres Protein (Molekulargewicht jeweils 47.000 ± 10%)
Temperaturoptimum 45 °C
pH-Optimum pH = 8,0
K_{M}-Wert bei 30 °C und pH = 7,8 für Glutathion 6,8 mMol und für Glutathion-S-biman 1,0 mMol
vollständige Hemmung in Gegenwart von Ethylendiamintetraessigsäure (EDTA), reversibel nach Entfernung von EDTA und Zugabe von Schwermetallionen
Cofaktoren: Schwermetallionen
Schwermetalle im Sinne der Erfindung sind Metalle, deren Dichte größer ist als die Dichte von Eisen.
Beispiele solcher Schwermetallionen sind insbesondere die Kationen der Metalle Blei, Zinn, Wismut, Titan, Mangan, Kobalt, Nickel, Kupfer, Silber, Gold, Platin, Zink, Cadmium, Quecksilber, Uran, Arsen, Selen.

γ-Glutamylcysteintransferase ist erhältlich durch Extraktion pfanzlichen Materials. Grundsätzlich kommen als pflanzliches Material sowohl die niederen als auch die höheren Pflanzen in Betracht, da das erfindungsgemäße enzym ein Enzym des Primärstoffwechsels ist. Beispiele pflanzlichen Materials sind
Rauvolfia serpentina (Apocynaceae)
Asclepias syriaca (Asclepiadaceae)
Berberis stolonifera (Berberidaceae)
Beta vulgaris (Chenopodiaceae)
Silene cucubalus (Caryophyllaceae)
Helianthus annuus (Compositae)
Dioscorea composita (Dioscoreaceae)
Fumaria capreolata (Fumariaceae)
Agrostis tenuis (Graminaceae)
Iris pallida (Iridaceae)
Crotalaria cobalticola, Phaseolus vulgaris (Leguminosae)
Cissampelos mucronata (Menispermaceae)
Glaucium flavum (Papaveraceae)
Coptis japonica (Ranunculaceae)
Rosa canina (Rosaceae)
Morinda citrifolia (Rubiaceae)
Nicotiana tabacum (Solanaceae)
Viola calaminaria (Violaceae)
sowie:
Euglena gracilis, Fragillaria crotonensis, Navicula pelliculosa, Bumilleriopsis filiformis, Chlamydomonas reinhardii, Chlorella fusca, Monoraphidium minutum, Scenedesmus acutiformis, Stichococcus bacillaris, Sargassum muticum, Porphyridium cruentum (Phycophyta)
und
Amylostereum chailetti, Sarcosypha austriaca, Schizosaccharomyces pombe, Schizosaccharomyces versatilus, Sporobolomyces holsaticus (Mycophyta).

Vorzugsweise werden die höheren Pflanzen Silene cucubalus und Crotalana cobalticola, sowie Schizosaccharomyces pombe als Pilz verwendet.

Als pflanzliches Material werden vorzugsweise Zellkulturen der genannten Pflanzen herangezogen. Es können jedoch auch die differenzierten Pflanzen oder Pflanzenteile einschließlich der Wurzeln zur Gewinnung des erfindungsgemäßen Enzyms herangezogen werden.

Die Extraktion des pflanzlichen Materials wird nach Methoden durchgeführt, nach denen auch bereits bisher Enzyme aus Pflanzen extrahiert werden konnten. Üblicherweise wird zunächst so vorgegangen, daß die Zellstruktur der Pflanzen bzw. der entsprechenden Zellkulturen zerstört wird. Hierzu wird das pflanzliche Material zweckmäßigerweise eingefroren, z. B. bei der Temperatur des flüssigen Stickstoffs, ggf. zerkleinert und schließlich in einer wäßrigen Zubereitung vom pH 6 bis pH 10 aufgenommen. Als wäßrige Zubereitungen werden insbesondere für den genannten pH-Bereich geeignete Pufferlösungen, beispielsweise Phosphatpuffer, verwendet. Gegenbenenfalls nach Abtrennung nicht aufgeschlossenen Pflanzenmaterials kann wahlweise eine Konzentrierung der Proteine durch Proteinfällung und anschließende Gelfiltration erfolgen. Die Proteinfällung wird durch Zugabe von Elektrolyt (insbesondere Ammoniumsulfat) vorgenommen. Die gefällten Proteine werden anschließend in einer wäßrigen Pufferlösung (pH = 6 bis 10) aufgenommen und einer Gelfiltration unterworfen. Durch die Gelfiltration wird sowohl eine Entsalzung als auch eine Auftrennung des vorliegenden Proteingemisches nach Maßgabe der Molekülgröße erreicht. Zur weiteren Aufarbeitung wird derjenige Teil des Eluats aus der Gelfiltration verwendet, der die Proteine vom Molgewicht > 40.000 enthält. Die nachfolgenden Reinigungsschritte umfassen die weitere chromatographische Trennung des Proteingemisches an hydrophober Säulenmatrix (z.B. Phenylsepharose, Pharmacia, Freiburg), an Hydroxyapatit (Biorad) und an Ionenaustauschern (z.B. Diethylaminoethylcellulose). Die Auswahl an Eluat wird dabei stets nach Maßgabe der Enzymaktivitätskontrollen getroffen. Falls erwünscht, können auch andere oder zusätzliche Trennungsmethoden angewendet werden, wie beispielsweise Elektrophorese, isoelektrische Fokussierung und dergleichen.

Die vorstehend beschriebenen Aufarbeitungsprozeduren werden im wäßrigen System, insbesondere in Pufferlösungen vom pH 6 bis 10, insbesondere pH 7 bis 9, durchgeführt, bei Temperaturen von vorzugsweise 4 °C bis 15 °C.

Nach dem erfindungsgemäßen Verfahren werden wäßrige oder ggf. gefriergetrocknete Zubereitungen erhalten, die eine enzymatische Aktivität entsprechend einem Gehalt an γ-Glutamylcysteinransferase aufweisen.

Gegenstand der Erfindung sind somit enzymatisch aktive Zusammensetzungen, die γ-Glutamylcysteintransferase enthalten.

Das erfindungsgemäße Enzym kann oftmals mit Vorteil in immobilisiertem Zustand eingesetzt werden. Hierzu wird das Enzym in an sich bekannter Weise an Trägermaterialien physisorbiert oder chemisorbiert. Beispiele für Trägermaterialien sind Alginate, funktionelle Gruppen aufweisende Agarosen, Zellulose, Polyacrylharze (mit z.B. Oxirangruppen), Gläser, Silikate.

Das erfindungsgemäße Enzym katalysiert den Transfer von γ-Glutamylcystein bzw. von γ-Glutamylcystein-S-derivaten.

Gegenstand der Erfindung ist somit ferner ein Verfahren zur enzymatischen Polymerisation, das dadurch gekennzeichnet ist, daß Verbindungen der allgemeinen Formel I

H₂N-CH(COOH)-(CH₂)₂-C(O)-NH-CH(CH₂-SR¹)-C(O)-X ,

wobei
- R¹: Wasserstoff-, Alkyl- oder Arylrest und
- X: eine Gruppierung der allgemeinen Formel
NHR²
mit R² gleich Wasserstoff-, Alkyl- oder Arylrest,
oder NH-CR(R³)-COOH
mit der Maßgabe, daß R³ den entsprechenden Rest aller natürlich vorkommender Aminosäuren der Formel NH₂CH(R³)COOH wiedergibt,
bedeuten,
in Gegenwart von γ-Glutamylcysteintransferase umgesetzt werden.

Vorzugsweise stehen R¹ für Wasserstoff-, 2,4-Dinitrophenyl- oder Bimanrest,
R² für Wasserstoff-, Carboxyethyl-, 4-Nitrophenyl- oder Naphthylrest und
R³ für Wasserstoff-, Hydroxyl-, Methyl- und Benzylrest.

Beispiele für erfindungsgemäß zu polymerisierende Verbindungen sind Glutathion, Homoglutathion und γ-Glutamylcysteinylamid.

Durch das erfindungsgemäße Verfahren werden Polymere der allgemeinen Formel II

[H₂N-CH(COOH)-(CH₂)₂-C(O)-NH-CH(CH₂-SR¹)-C(O)]ₙ-X

erhalten, wobei der Polymerisationsgrad n innerhalb des Bereiches von 2 bis 20 liegt.

Beispiele solcher Polymere sind Phytochelatin mit R¹=H und X=NHCH₂COOH; Homo-Photochelatin mit R¹=H und X=NHCH₂CH₂OH, Poly-(Glutamylcystein) mit R¹=H und X=OH und Poly-(glutamylcystein)amid mit R¹=H und X=NH₂.

Die Reaktionstemperaturen betragen vorzugsweise 10 bis 70 °C, insbesondere 25 bis 50 °C.

Vorzugsweise wird so vorgegangen, daß eine wäßrige Zubereitung von Substanzen der allgemeinen Formel I vom pH 6 bis 10, insbesondere 8,0, vorzugsweise in Gegenwart von oben genannten Schwermetallionen einer Konzentration von 0,1 µmol/l bis 10 mmol/l, insbesondere 10 bis 500 µ mol/l, mit einer wäßrigen Enzymzubereitung oder einer Zubereitung, in der das Enzym in immobilisiertem Zustand vorliegt, versetzt und gerührt wird.

Die Menge an eingesetztem Enzym, bezogen auf die Substratmenge, ist an sich unkritisch. Sie wird nach Maßgabe des erwünschten Produktumsatzes eingestellt. Üblicherweise werden pro 1 Mol einzusetzendes Substrat Enzymmengen im Bereich von 1 bis 50 g, insbesondere 15 bis 20 g eingesetzt.

Die Verfolgung des Reaktionsverlaufes erfolgt beispielsweise durch Hochdruckchromatographie mit underivatisierten bzw. derivatisierten Reaktionsprodukten.

Die Umsetzungsprodukte gemäß der allgemeinen Formel II finden Verwendung zur Schwermetallentgiftung.

### Beispiel 1

### Herstellung von γ-Glutamylcysteintransferase

500 g mit flüssigem Stickstoff eingefrorener Zellkultur aus Silene cucubalus wurden 20 Minuten in 250 ml einer wäßrigen Trihydroxymethylaminomethan-HCl-Pufferlösung (50 millimolar, pH = 8,5) mit 10mM Mercaptoethanol gerührt. Das entstandene Zellhomogenat wurde anschließend abfiltriert und zentrifugiert. Dem Überstand wurden 86 g/l festen Ammoniumsulfats zugegeben. Die dabei entstandene Proteinfällung wurde abzentrifugiert und die Proteine des Überstandes an eines Phenyl-Sepharose-Säule (2,5 x 20 cm, Pharmacia, Freiburg) gebunden. Die Säule wurde mit 0,2 l Ammoniumsulfatlösung (10mM Tris-HCl, pH = 7,8, 86g Ammoniumsulfat pro Liter,10mM Mercaptoethanol) gewaschen und anschließend gebundenes Protein mit einer Lösung der Zusammensetzung 10mM Tris-HCl, 10mM Mercaptoethanol und 10% Ethylenglycol (pH = 7,8) eluiert. Das Eluat wurde auf eine Hydroxyapatit-Säule (1,5x11,5cm; Biorad, München) gepumpt, die mit einer Lösung aus 10 mM Kaliumphosphatpuffer, pH = 7,8, 10 mM Mercaptoethanol, 10 mM NaCl und 0,5 mM MgCl₂ äquilibriert war. Die Säule wurde mit 100ml des genannten Puffers gewaschen und anschließend die γ-Glutamylcysteintransferase durch einen Stufengradienten der Zusammensetzung 20 bis 100 mM Kaliumphosphat und 10 mM Mercaptoethanol eluiert. Die aktive Fraktion dieser Elution wurde auf einen Ionenaustauscher ((Anionen-)MonoQ, Pharmacia, Freiburg) aufgetragen, nachdem das Kaliumphosphat durch Ultrafiltration (Amicon, Düren) und waschen des Retentats mit einem Puffer aus 20 mM Tris-HCl-Puffer, pH = 8,5, 10 mM NaCl, 10 mM Mercaptoethanol und 0,5 mM MgCl₂ auf mindestens 1/10 der anfänglichen Konzentration verdünnt worden war. Es wurde mit einem NaCl-Gradienten 10-350 mM NaCl in o.g. Puffer eluiert. Die aktive Fraktion dieser Elution wurde durch Zugabe einer 50-%igen Ammoniumsulfatlösung in 10 mM Tris-HCl, pH = 7,8, auf 10-Gew.-% Ammoniumsulfat eingestellt, auf eine Phenyl-Suparose-Säule (Pharmacia, Freiburg) gepumpt und mit einem linearen Gradienten, bestehend aus A 86 g Ammoniumsulfat pro Liter in 10 mM Tris-HCl, 10 mM Mercaptoethanol, 10 mM NaCl und 0,5 mM MgCl₂, sowie B 10 mM Tris-HCl, 10 mM Mercaptoethanol, 10 mM NaCl, 0,5 mM MgCl₂ und 10% Ethylenglycol, eluiert. Die aktive Fraktion dieser Elution wurde einer Gelfiltration an SW 3000 (LKB, jetzt Pharmacia, Freiburg) in 50 mM β-Alanin, 10 mM Histidin, pH 6,6, 10 mM Mercaptoethanol und 0,5 mM MgCl₂ unterzogen. Die aktive Fraktion zeigte eine Aktivität von 1 nkat/mg Protein. Die Ausbeute betrug 10%.

Alternativ kann nach der Hydroxyapatitsäule folgender Reinigungsgang eingesetzt werden:
Die aktiven Fraktionen wurden mit 10% Glycerin versetzt, auf eine Aca 34-Säule (Pharmacia, Freiburg) aufgetragen und mit 10 mM Tris-HCl-Puffer pH 7,8 und 10mM Mercaptoethanol eluiert. Die aktiven Fraktionen wurden auf einen Anionenaustauscher QAE FF (Pharmacia, Freiburg) aufgegeben und mit einem KCl-Gradienten, 150-400 mM KCl eluiert. Die aktiven Fraktionen wurden durch Zugabe von pulverisiertem Ammoniumsulfat auf 10-Gew.-% Ammoniumsulfat eingestellt, auf eine Phenylsuperose-Säule (Pharmacia, Freiburg) gepumpt und mit einem linearen Gradienten, bestehend auf A 85 g Ammoniumsulfat pro Liter in 10 mM Tris-HCl-Puffer und 10 mM Mercaptoethanol sowie B 10 mM Tris-HCl-Puffer und 10 mM Mercaptoethanol eluiert. Die aktiven Fraktionen wurden gegen 10 L Kaliumphosphatpuffer pH 8,0 (25mM) dialysiert und anschließend auf eine Chelating Sepharose-Säule (Pharmacia, Freiburg) aufgetragen die mit Zn²⁺ beladen war. Die Elution erfolgte mit 50mM Imidazol in 25mM Kaliumphosphatpuffer pH 8,0. Die aktive Fraktion zeigte eine spezifische Aktivität von 1 nkat/mg Protein.

### Beispiel 2

### Herstellung von immobilisierter γ-Glutamylcysteintransferase

6 ml einer Enzymzubereitung von γ-Glutamylcysteintransferase in einer wäßrigen Tris-hydroxy-aminomethan/HCl-Pufferlösung (10 mM Mercaptoethanol, 10 mM NaCl, 0,1 molar, pH = 7,8) mit einer Enzymaktivität von 1,2 nkat/mg und einem Proteingehalt von 1,02 mg/ml wurden in 50 ml einer 5 Gew.-%-igen, wäßrigen Alginatlösung unter Rühren eingetragen. Die enzymhaltige Alginatlösung wurde anschließend bei 5 °C unter Rühren in 500 ml einer 0,1 molaren, wäßrigen Calciumchloridlösung getropft.
Es wurde ein kugelförmiges Produkt erhalten von immobilisiertem Enzym an Calciumalginat, das noch mit oben genanntem Puffer gewaschen wurde. Das Präparat wies eine Enzymaktivität von 0,32 nkat/mg Protein auf.

### Beispiel 3

### Enzymatische Polymerisation von Glutathion

50 ml einer Enzymzubereitung von γ-Glutamylcysteintransferase in einer wäßrigen Lösung von Tris-HCl (0,1 molar, pH 7,8), 15 mM Glutathion, 10 mM NaCl mit einer Enzymaktivität von 1,1 nkat/mg Protein und einem Proteingehalt von 0,26 mg/ml wurden kontinuierlich über eine Stunde mit 20 µmol einer 10 mM Cd²⁺-Lösung versetzt.
Nach einer Stunde wurden 15 mg Phytochelatine mit einem Cd²⁺-Gehalt von 18,2 Gew.-% erhalten. Die Entfernung des Cd²⁺-Bestandteils erfolgte durch Fällung mit gasförmigem Schwefelwasserstoff.

## Patentansprüche

1. Transferase Enzym, das den Transfer von - Glutamylcysteinresten aus Glutathion, unter Bildung von Glutathionpolymeren (Phytochelatine), katalysiert.

2. Enzym nach Anspruch 1, gekennzeichnet durch folgende Parameter:
a) Molgewicht 95.000 ± 10%, bestimmt durch Gelfiltration; Enzym vorliegend als dimeres Protein (Monomer 47.000 ± 10%)
b) Temperaturoptimum 45°C
c) pH-Optimum = 8,0
d) K_{M}-Wert bei 30°C und pH = 7,8 für Glutathion 6,8 mMol und für Glutathion-S-biman 1,0 mMol
e) vollständige Hemmung in Gegenwart von Ethylendiamintetraessigsäure (EDTA), reversibel nach Entfernung von EDTA und Zugabe con Schwermetallionen
f) Cofaktoren: Schwermetallionen

3. Enzym nach Anspruch 1, erhältlich durch Extraktion pflanzlichen Materials aus der Familie der Apocynaceae, Asclepiadaceae, Berberidaceae, Chenopodiaceae, Caryophyllaceae, Compositae, Dioscoreaceae, Fumariaceae, Graminaceae, Iridaceae, Leguminosae, Menispermaceae, Papaveraceae, Ranunculaceae, Rubiaceae, Solanaceae, Violaceae, sowie durch Extraktion der Gewebe von Algen (Phycophyta) und Pilzen (Mycophyta).

4. Enzymatisch aktive Zusammensetzungen, die das Enzym nach Ansprüchen 1-3 enthalten.

5. Immobilisiertes Enzym nach Ansprüchen 1-3.

6. Verfahren zur Herstellung des Enzyms nach Ansprüchen 1-3, dadurch gekennzeichnet, dass pflanzliches Material aus der Familie der Apocynaceae, Asclepiadaceae, Berberidaceae, Chenopodiaceae, Caryophyllaceae, Compositae, Dioscoreaceae, Fumariaceae, Graminaceae, Iridaceae, Leguminosae, Menispermaceae, Papaveraceae, Ranunculaceae, Rubiaceae, Solanaceae, Violaceae, extrahiert wird, sowie durch Extraktion der Gewebe von Algen (Phycophyta) und Pilzen (Mycophyta).

7. Verfahren zur enzymatischen Polymerisation, dadurch gekennzeichnet, dass Verbindungen der allgemeinen Formel I
H₂N-CH(COOH)-(CH₂)₂-C(O)-NH-CH(CH₂-SR¹)-C(O)-X
wobei
R¹ Wasserstoff-, Alkyl- oder Arylrest und
X eine Gruppierung der allgemeine Formel NHR², mit R² gleich Wasserstoff-, Alkyl- oder Arylrest, oder NH-CH(R³)-COOH
mit der Maßgabe, daß R³ den entsprechenden Rest aller natürlich vorkommender Aminosäuren der Formel NH₂CH(R³)COOH wiedergibt,
bedeuten,
in Gegenwart des Enzyms nach Ansprüchen 1-3 umgesetzt werden.

## Claims

1. A transferase enzyme which catalyses the transfer of the γ-glutamylcysteine fragment from glutathione, to form glutathione polymers (phytochelatins).

2. An enzyme according to Claim 1, characterised by the following parameters:
a) molecular weight 95,000 ± 10%, determined by gel filtration; enzyme present as dimeric protein (monomer 47,000 ± 10%)
b) optimum temperature 45°C
c) optimum pH = 8.0
d) K_{M} value at 30°C and pH = 7.8 for glutathione 6.8 mMol and for glutathione-S-dimer 1.0 mMol
e) complete inhibition in the presence of ethylenediamine tetraacetic acid (EDTA), reversible after removal of EDTA and addition of heavy-metal ions
f) cofactors: heavy-metal ions

3. An enzyme according to Claim 1, obtainable by extraction of vegetable material from the family of Apocynaceae, Asclepiadaceae, Berberidaceae, Chenopodiaceae, Caryophyllaceae, Compositae, Dioscoreaceae, Fumariaceae, Graminaceae, Iridaceae, Leguminosae, Menispermaceae, Papaveraceae, Ranunculaceae, Rubiaceae, Solanaceae, Violaceae, and by extraction of the tissue of algae (phycophyta) and fungi (mycophyta).

4. Enzymatically active compositions containing the enzyme according to Claims 1-3.

5. An immobilized enzyme according to Claims 1-3.

6. A process for the production of the enzyme according to Claims 1-3, characterised in that vegetable material is extracted from the family of Apocynaceae, Asclepiadaceae, Berberidaceae, Chenopodiaceae, Caryophyllaceae, Compositae, Dioscoreaceae, Fumariaceae, Graminaceae, Iridaceae, Leguminosae, Menispermaceae, Papaveraceae, Ranunculaceae, Rubiaceae, Solanaceae, Violaceae, and by extraction of the tissue of algae (phycophyta) and fungi (mycophyta).

7. A process for enzymatic polymerisation, characterised in that compounds of the general formula I
H₂N-CH(COOH)-(CH₂)₂-C(O)-NH-CH(CH₂-SR¹)-C(O)-X,
wherein
R¹ denotes hydrogen residue, alkyl residue or aryl residue, and
X denotes a grouping of the general formula NHR², with R² equal to hydrogen residue, alkyl residue or aryl residue, or NH-CH(R³)-COOH, with the proviso that R³ describes the appropriate residue of all naturally occurring amino acids of the formula NH₂CH(R³)COOH,
are converted in the presence of the enzyme according to Claims 1-3.

## Revendications

1. Enzyme de type transférase qui catalyse le transfert du résidu γ-glutamylcystéine du glutathion, accompagné de la formation d'un polymère de glutathion (Phytochélatine).

2. Enzyme selon la revendication 1, caractérisée par les paramètres suivants :
a) un poids moléculaire, estimé par gel-filtration, de 95 000 ± 10% ; enzyme sous forme de protéine dimérique (Monomère 47 000 ± 10%)
b) température optimale de 45°C
c) pH optimal = 8,0
d) une valeur de K_{M}, à 30°C et à un pH = 7,8, de 6,8 mM pour le glutathion et de 1,0 mM pour le glutathion-S-biman
e) une inhibition complète en présence d'acide éthylène diaminetétracétique (EDTA), réversible après élimination de l'EDTA et addition d'un ion métallique lourd
f) cofacteur : un ion métallique lourd

3. Enzyme selon la revendication 1 qui peut être obtenue par extraction à partir d'une plante appartenant à la famille des Apocynacées, des Asclepiadacées, des Berberidées, des Chénopodiacées, des Caryophyllacées, des Composacées, des Dioscoréacées, des Fumariacées, des Graminées, des Iridées, des Légumineuses, des Ménispermacées, des Papavéracées, des Renonculacées, des Rubiacées, des Solanacées, des Violacées ainsi que par extraction à partir d'un tissu d'algues (phycophytes) et de champignons (mycophytes).

4. Compositions présentant une activité enzymatique qui comprennent une enzyme selon les revendications 1 à 3.

5. Enzyme selon les revendications 1 à 3 sous forme immobilisée.

6. Procédé de production des enzymes selon les revendications 1 à 3, caractérisé en ce qu'une plante appartenant à la famille des Apocynacées, des Asclepiadacées, des Berberidées, des Chénopodiacées, des Caryophyllacées, des Composacées, des Dioscoréacées, des Fumariacées, des Graminées, des Iridées, des Légumineuses, des Ménispermacées, des Papavéracées, des Renonculacées, des Rubiacées, des Solanacées, des Violacées est soumise à une opération d'extraction, ou par l'extraction d'un tissu d'algue (phycophytes) et de champignons (mycophytes).

7. Procédé de polymérisation enzymatique, caractérisé en ce que des composés de formule I
H₂N-CH(COOH)-(CH₂)₂-C(O)-NH-CH(CH₂-SR¹⁾-C(O)-X
dans laquelle R¹ représente un atome d'hydrogène, un groupe alkyle ou un groupe aryle et X représente un groupement de formule générale NHR², où R² est un atome d'hydrogène, un groupe alkyle ou aryle ou un groupement NH-CH(R³)-COOH,
sous réserve que le groupe R³ reproduit le résidu correspondant de tous les acides aminés de formule NH-CH(R³)-COOH qui existent dans la nature, sont transformés en présence de l'enzyme selon les revendications 1 à 3.
